# FASCICULE DE BREVET EUROPEEN

(11) **EP 1 119 305 B1**
(45) Date de publication et mention de la délivrance du brevet: **18.05.2005**
(21) Numéro de dépôt: 99970307.7
(22) Date de dépôt: 07.10.1999
(51) Int. Cl.: A61B 17/70

(54) **SYSTEME D'OSTEOSYNTHESE RACHIDIENNE A RIGIDITE AMELIOREE**
SPINALES OSTEOSYNTHESESYSTEM MIT VERBESSERTER STABILITÄT
SPINAL OSTEOSYNTHESIS SYSTEM WITH IMPROVED STABILITY

(30) Priorité: 09.10.1998 FR 9812662
(43) Date de publication de la demande: 01.08.2001
(73) Titulaire: Dimso (Distribution Médicale du Sud-Ouest), 33610 Cestas (FR)
(72) Inventeur: CONCHY, Frédéric, F-33650 Saint-Médard-d'Eyrans (FR); ASSAKER, Richard, B-7540 Kain (BE)
(74) Mandataire: Le Forestier, Eric
(86) Numéro de dépôt international: PCT/FR1999/002402
(87) Numéro de publication internationale: WO 2000/021447

(56) Documents cités:
- DE-A- 4 433 360
- FR-A- 2 731 344
- US-A- 5 613 968

## Description

L'invention concerne les systèmes d'ostéosynthèse rachidienne notamment pour fixation antérieure.

On connaît des systèmes d'ostéosynthèse rachidienne pour fixation antérieure dans lesquels l'élément de liaison est constitué par une plaque et d'autres dans lesquels il est constitué par une tige. Du fait de leur encombrement, les systèmes à plaque sont difficilement, voire pas du tout utilisables par voie endoscopique. De plus, leur taille limitée (longueur) ne permet de les utiliser que pour de simples corporectomies concernant une seule vertèbre, voire deux. Il est impossible de traiter une scoliose avec ce genre de système. Enfin, une plaque est difficilement adaptable à la morphologie des vertèbre où elle est ancrée. Par ailleurs, les systèmes à tige comprennent généralement des connecteurs assez volumineux qui ne sont pas toujours utilisables par voie endoscopique.

On connaît par ailleurs d'après le document FR-2 731 344, qui a pour homologue le WO-96/27340, un dispositif d'ostéosynthèse rachidienne comprenant un connecteur à deux branches pouvant serrer entre elles une tige de liaison, les branches étant aptes à être enfilées sur une vis pédiculaire vertébrale. La tige est reçue entre les branches avec interposition d'une bague fendue à face sphérique enfilée sur la tige pour régler l'orientation angulaire du connecteur par rapport à la tige avant serrage. Toutefois, ce dispositif, bien adapté pour une fixation en partie postérieure du rachis sur les pédicules vertébraux, ne fournit pas toujours une rigidité suffisante en vue d'une fixation en partie antérieure du rachis.

Le document US-5 613 968 divulgue un système d'ostéosynthèse dans lequel un connecteur reçoit deux éléments de liaison allongés.

Un but de l'invention est de fournir un système d'ostéosynthèse rachidienne différent, adapté pour une fixation antérieure, facile à monter, assurant une bonne rigidité du système sur le rachis et compatible avec un montage par voie endoscopique.

En vue de la réalisation de ce but, on prévoit selon l'invention un système d'ostéosynthèse rachidienne selon la revendication 1.

Ainsi, la présence des deux éléments de liaison confère au système une très grande rigidité, sans compliquer son assemblage, sans accroître le volume de ses différentes pièces (ce qui le rend compatible avec un montage par voie endoscopique), et tout en conservant la possibilité de réglage de la position angulaire du connecteur par rapport au premier élément de liaison. Le système selon l'invention ne requiert pas un cintrage identique sur les deux éléments de liaison. De plus, le nombre de connecteurs peut rester peu élevé.

De plus, la forme du deuxième élément de liaison commande la position angulaire relative des connecteurs fixés à celui-ci. On peut donc prévoir et choisir à l'avance cette position angulaire suivant la courbure préalable que l'on donne à cet élément de liaison, à la fabrication ou mieux lors de l'intervention chirurgicale.

Avantageusement, le deuxième élément de liaison présente une résistance au cintrage plus faible que le premier élément de liaison.

Ainsi, le premier élément de liaison a principalement une fonction de support des connecteurs et le deuxième élément de liaison a principalement une fonction de positionnement angulaire relatif des connecteurs.

Avantageusement, le connecteur comporte deux branches aptes à serrer au moins l'un des deux éléments de liaison.

Avantageusement, les branches présentent des premières extrémités par lesquelles elles sont reliées l'une à l'autre et des deuxièmes extrémités libres.

Ainsi, on simplifie la fabrication du connecteur. De plus, en choisissant convenablement la souplesse du connecteur et/ou le diamètre du deuxième élément de liaison, on peut introduire cet élément latéralement entre les extrémités libres des branches, ce qui facilite le montage.

Avantageusement, le connecteur est apte à recevoir le deuxième élément de liaison par insertion latérale entre les extrémités libres.

Avantageusement, les branches sont aptes à serrer simultanément les deux éléments de liaison.

Avantageusement, l'organe d'ancrage comprend une vis vertébrale, au moins une première des deux branches étant apte à être engagée sur la vis.

Avantageusement, la vis vertébrale présente un orifice fileté, le système comprenant une vis de serrage apte à constituer une liaison vis-écrou avec cet orifice et à prendre appui sur une des branches pour serrer les branches.

Avantageusement, le système comprend une deuxième vis vertébrale, la première branche étant apte à être engagée simultanément sur les deux vis vertébrales.

Avantageusement, le système est agencé de sorte que le deuxième élément de liaison, lorsqu'il est fixé au connecteur, s'étend dans une trajectoire de la deuxième vis vertébrale pour son désengagement du connecteur.

Ainsi, on prévient tout début de sortie intempestive de la deuxième vis.

Avantageusement, le système comprend une bague apte à être enfilée sur le premier élément de liaison et reçue entre les branches pour choisir la position angulaire du premier élément de liaison avant serrage des branches.

D'autres caractéristiques et avantages de l'invention apparaîtront encore dans la description suivante d'un mode préféré de réalisation donné à titre d'exemple non limitatif. Aux dessins annexés :
- la figure 1 est une vue en perspective du système selon l'invention ;
- la figure 2 est une vue partielle en perspective éclatée du système de la figure 1 ;
- les figures 3 et 4 sont deux vues en perspective, respectivement de dessus et de dessous, montrant un des connecteurs du système de la figure 1 ;
- la figure 5 est une vue, moitié en élévation et moitié en coupe axiale, d'une bague du système de la figure 1 ;
- la figure 6 est une vue pour partie de dessus et pour partie en coupe du connecteur de la figure 3 recevant la première tige ;
- la figure 7 est une vue partielle en perspective montrant la tête de la vis principale ; et
- la figure 8 montre le système de la figure 1 fixé sur des vertèbres.

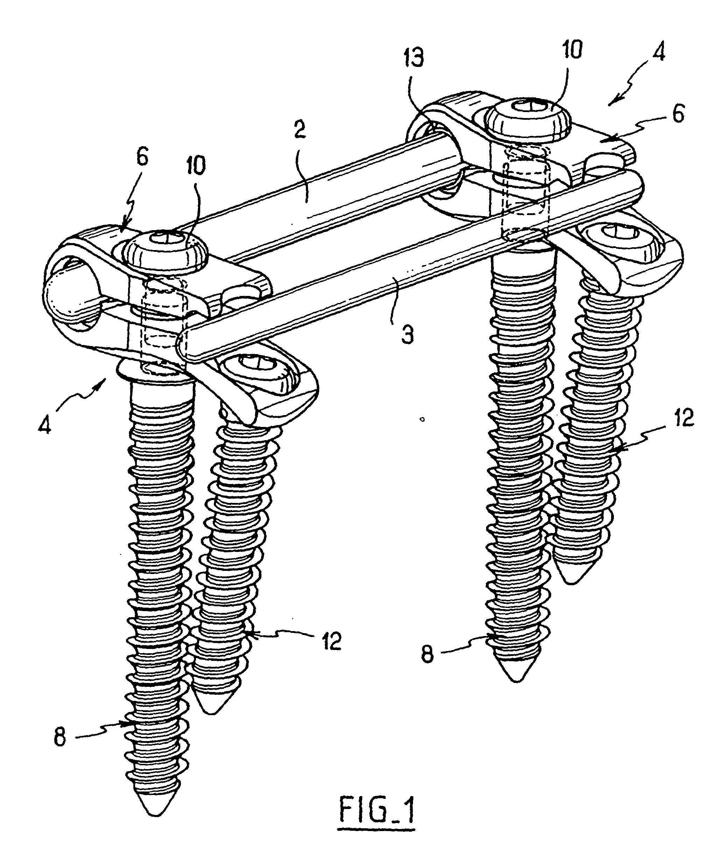

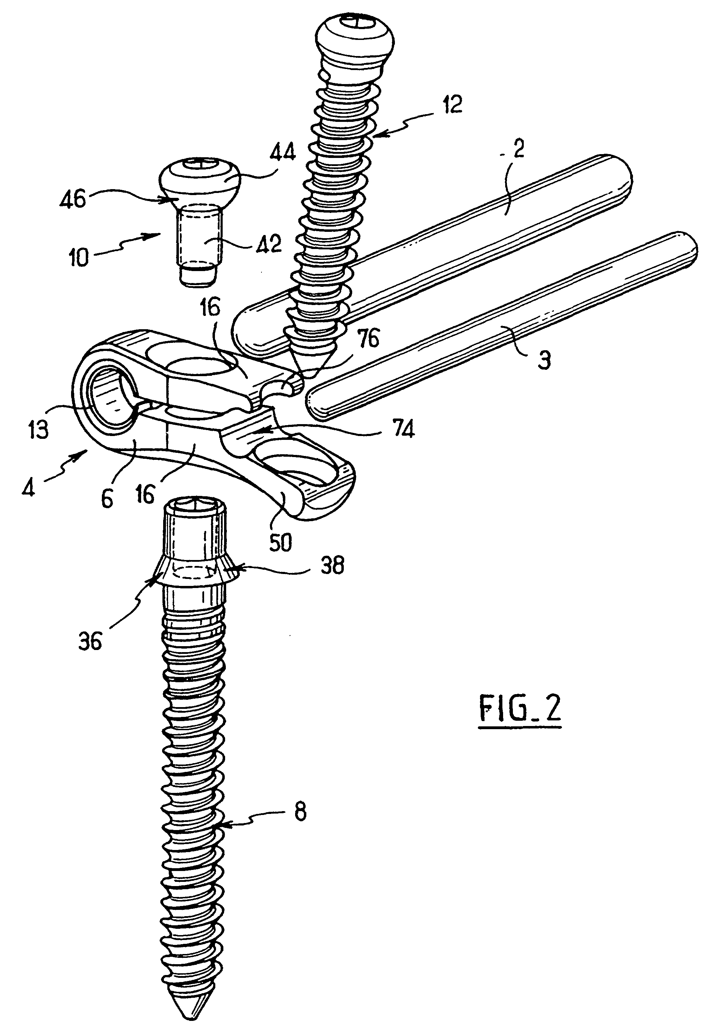

En référence aux figures 1 et 2, le système selon l'invention comprend dans le présent mode de réalisation une première tige de liaison allongée 2, ou tige principale, à section circulaire, une deuxième tige de liaison allongée 3 ou tige secondaire à section circulaire, et plusieurs sous-ensembles 4 à connecteur aptes à être fixés simultanément à celles-ci. Chacun de ces sous-ensembles, dont deux seulement sont visibles sur la figure 1 et un seul est visible sur la figure 2, comprend un connecteur 6, une première vis vertébrale ou vis principale 8, une vis de serrage 10, une deuxième vis vertébrale ou vis secondaire 12, et une bague 13.

Dans le présent exemple, le connecteur 6 est d'une seule pièce. Les différentes parties du système sont en métal biocompatible.

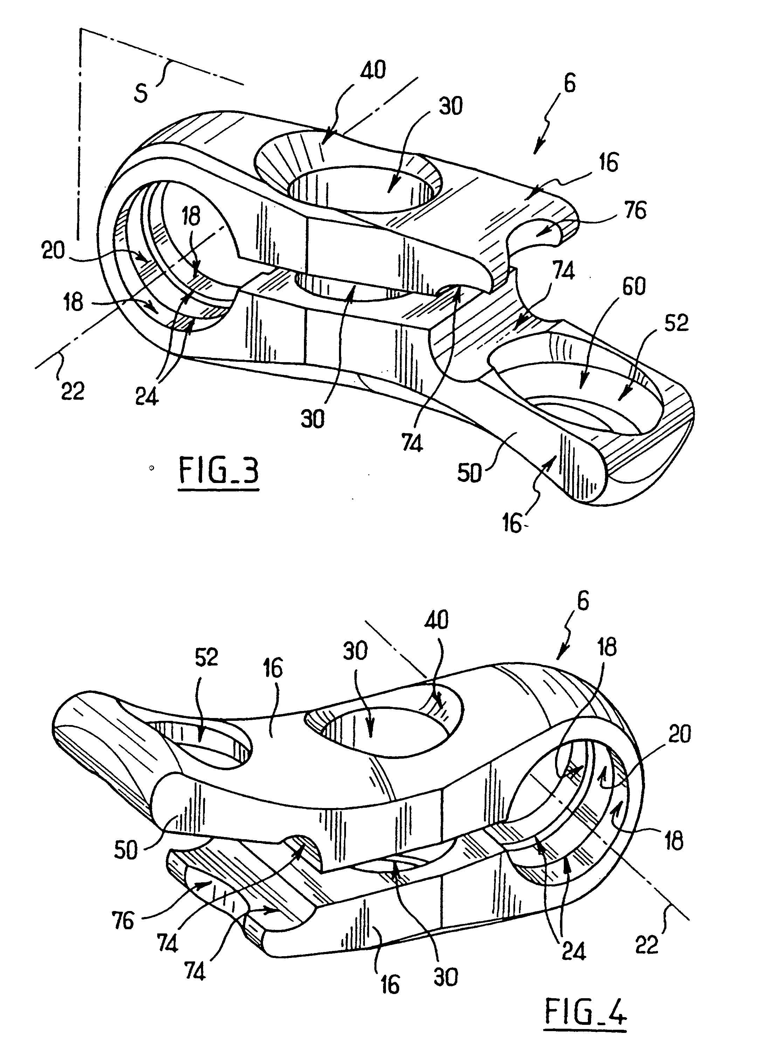

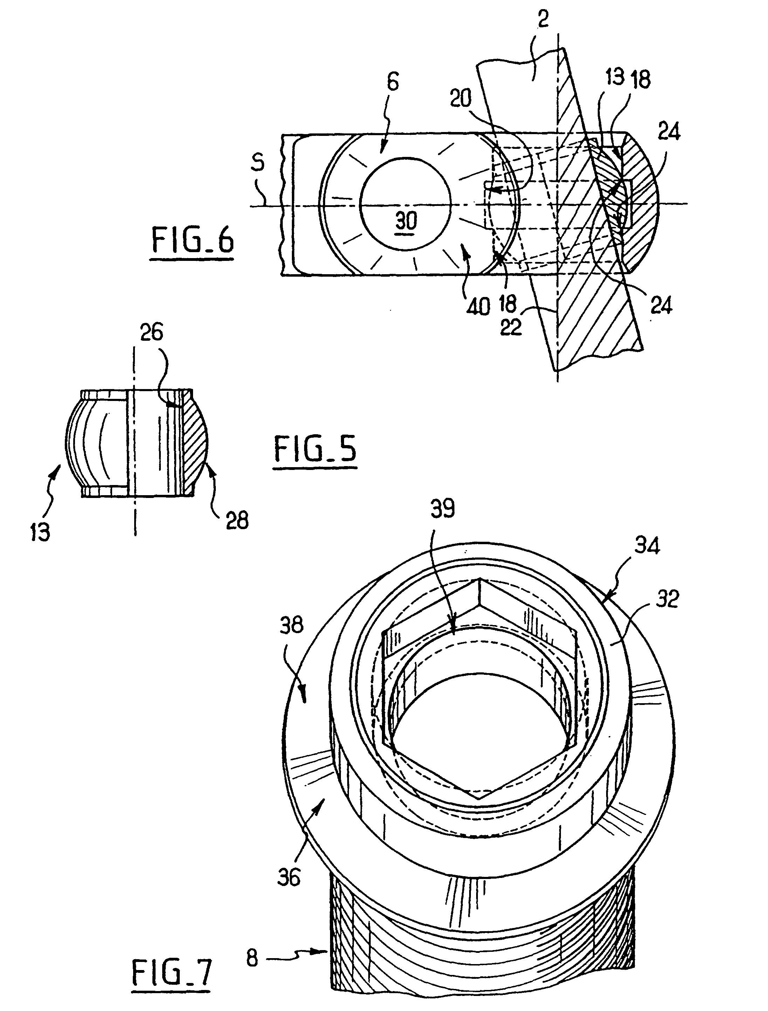

En référence aux figures 3 et 4, le connecteur 6 comporte deux branches 16 s'étendant en regard et à distance l'une de l'autre en donnant au connecteur un profil général en "U". Le connecteur 6 comporte un plan de symétrie S visible sur les figures 3 et 6, perpendiculaire à la largeur des branches 16 et parallèle à leur longueur. Ainsi, chaque branche présente une première extrémité et une deuxième extrémité. Les branches sont reliées l'une à l'autre par leurs premières extrémités. Les deuxièmes extrémités sont des extrémités terminales libres. En référence à la figure 6, au niveau de la naissance des branches 16, c'est-à-dire de leur première extrémité, le connecteur présente deux faces internes cylindriques coaxiales 18, 20 d'axe 22° perpendiculaire au plan S et de rayons différents. La face 20 de plus grand rayon est en deux parties disjointes, et s'étend de part et d'autre de la face 18 de plus petit rayon, laquelle est traversée par le plan S. Les deux faces 18, 20 forment à leurs jonctions deux arêtes circulaires 24 d'axe 22.

En référence aux figures 5 et 6, la bague 13 présente une face interne cylindrique 26 et une face externe sphérique 28 coaxiales. La face interne cylindrique 26 a un rayon environ égal à celui de la tige principale 2 de sorte que la bague 13, fendue sur un côté suivant son axe, peut être reçue par ajustement glissant sur la tige principale 2. Par ailleurs, la bague 13 peut être logée entre les branches 16 en regard des faces cylindriques 18, 20. La face externe sphérique 28 de la bague a un rayon adapté pour que dans cette position, les arêtes 24 du connecteur 6 soient en contact linéaire avec la face externe sphérique 28 de la bague 13 et lui servent d'appuis. Dans cette position, avant serrage des branchès 16, la position angulaire de la tige principale 2 enfilée dans la bague 13 peut être réglée dans deux plans perpendiculaires entre eux sur une amplitude par exemple de 15° de part et d'autre d'une position moyenne de la tige dans laquelle la tige est perpendiculaire au plan S.

En référence aux figures 3 et 4, les branches 16 présentent deux évidements cylindriques lisses respectifs qui sont en l'espèce des orifices traversants 30 s'étendant coaxialement en regard l'un de l'autre. La vis principale 8 est une vis vertébrale bicorticale et présente un corps fileté à cette fin de façon connue en soi. En référence à la figure 7, elle présente une tête 32 ayant une face externe cylindrique lisse 34. A la jonction entre la tête et le corps, la vis comporte une collerette annulaire 36 ayant une face inférieure plane perpendiculaire à un axe longitudinal de la vis, et une face supérieure tronconique 38 avec la section la plus étroite du tronc de cône située du côté de la tête 32 de la vis. La tête 32 présente un orifice fileté 39 coaxial au corps de la vis et, ménagée dans la face filetée de l'orifice 39, une forme non circulaire telle qu'une empreinte hexagonale à six pans. La vis de serrage 10 comporte un corps fileté 42 apte à former une liaison vis-écrou avec cet orifice 39, et une tête de vis 44 dans laquelle est formée une empreinte hexagonale à six pans. La tête 44 a une face externe inférieure sphérique convexe 46 dont la section la plus étroite est située vers la pointe de la vis.

L'une des branches 16 du connecteur 6, que pour la clarté nous appellerons ici branche inférieure, présente un prolongement 50 s'étendant en direction opposée aux faces cylindriques 18, 20 du connecteur. Il s'agit ici de la branche destinée à être adjacente à la vertèbre. Ce prolongement présente la deuxième extrémité, libre, de la branche. Les deux branches 16 sont aptes à être enfilées simultanément sur la tête 32 de la vis principale 8 introduite dans les orifices 30 à partir de la branche inférieure contre laquelle la face supérieure 38 de la collerette 36 vient en butée. On introduit ensuite la vis de serrage 10 dans la tête 32 de la vis principale 8 à partir de la branche supérieure 16. Le serrage de la vis de serrage 10 dans la tête 32 de la vis principale 8 provoque le rapprochement des branches 16 l'une de l'autre et, entre autres, le blocage par friction de la première tige 2 dans la position choisie par rapport au connecteur 6.

L'orifice 30 de la branche inférieure 16 a un bord inférieur, opposé à la branche supérieure et destiné à être du côté de la vertèbre, présentant une empreinte sphérique concave 40 destinée à venir en contact avec la face supérieure 38 de la collerette 36 pour réaliser par friction un blocage à rotation du connecteur 6 par rapport à l'axe de la vis principale 8. L'orifice 30 de la branche supérieure 16 a un bord supérieur, opposé à la branche inférieure et destiné à être opposé à la vertèbre, présentant une empreinte sphérique concave 40 destinée à venir en contact avec la face inférieure sphérique convexe 46 de la tête 44 de la vis de serrage 10 et permettant de fixer celle-ci ainsi que la vis principale 8 en réglant l'orientation angulaire de la vis principale 8 par rapport au connecteur.

Le prolongement 50 présente un évidement sous la forme d'un orifice traversant 52. La branche inférieure 16 est courbée au niveau du prolongement 50 dans une direction opposée à la branche supérieure 16 de sorte que les axes des orifices 30 et 52 qu'elle porte ne sont pas tout à fait parallèles. La vis secondaire 12 est une vis vertébrale, ici monocorticale, présentant un corps fileté et une tête 56 à face inférieure sphérique convexe 58 dont la section la plus étroite se trouve du côté du corps. Sa tête présente une empreinte à six pans. L'orifice 52 du prolongement a un bord supérieur 60, orienté du côté de l'autre branche 16 et destiné à être opposé à la vertèbre, présentant une forme sphérique concave ou de préférence tronconique 60, destinée à venir en contact avec la face inférieure sphérique convexe 58 de la tête 56 de la vis secondaire 12 en permettant de régler l'orientation angulaire de cette vis par rapport au connecteur 6.

On retrouvera dans les documents FR-2 731 344 et WO-96/27340 certaines des caractéristiques du connecteur 6 qui n'ont pas été développées ici en grand détail.

La branche inférieure 16 peut être cintrée pour accentuer ou réduire sa courbure en vue de mieux s'adapter à la forme de la partie antérieure de sa vertèbre de destination. Une fois cintrée, cette branche 16 n'est plus sollicitée en flexion puisqu'elle est fixée à la vertèbre par deux vis 8, 12 sur sa longueur. Les deux vis principale 8 et secondaire 12 sont autotaraudeuses et comportent des filetages à os.

Dans une variante de réalisation, la vis principale 8 ne présente pas une empreinte à six pans dans son orifice fileté 39, mais la collerette 36 a une forme hexagonale ou présente deux méplats parallèles et diamétralement opposés l'un à l'autre pouvant coopérer avec une clé de serrage pour mettre cette vis 8 en rotation par rapport au connecteur 6.

Les deux tiges de liaison 2, 3 ont chacune une forme rectiligne profilée à profil ici circulaire. La tige secondaire 3 a une section, transversalement à son axe longitudinal, de diamètre inférieur à celui de la tige principale 2. La tige principale 2 aura par exemple un diamètre de 6 mm. Par exemple, le diamètre de la tige secondaire 3 sera compris entre 30% et 80% du diamètre de la tige principale 2. Ce faible diamètre permet au chirurgien de choisir la courbure de la tige secondaire 3 qui correspond à celle de l'étage du rachis instrumenté. En revanche, puisque les bagues 13 permettent une angulation relative entre les deux connecteurs 6, la tige principale 2 n'a pas besoin d'être cintrée. Elle peut donc avoir un diamètre important pour être très robuste.

Les branches 16 du connecteur présentent des empreintes cylindriques ou mors 74 respectives, ménagées dans les faces des branches en regard l'une de l'autre. Les empreintes 74 s'étendent en regard l'une de l'autre et ont des axes parallèles entre eux, et perpendiculaires au plan de symétrie S.

Sur la branche supérieure 16, l'empreinte 74 s'étend à une extrémité libre de la branche de sorte que l'orifice 30 est interposé entre les faces 18, 20 d'une part, et l'empreinte 74, d'autre part. Sur la branche inférieure 16, l'empreinte 74 s'étend entre les deux orifices 30 et 52, à la naissance du prolongement 50. Elle est contiguë à l'orifice 52 de sorte qu'elle mord sur son bord 60.

La tige secondaire 3 est destinée à être reçue dans l'empreinte 74 de la branche inférieure 16 dans une position angulaire unique par rapport au connecteur, perpendiculaire au plan de symétrie S. Le connecteur et la tige sont dimensionnés de sorte que la tige secondaire peut être introduite latéralement entre les extrémités libres des branches (c'est-à-dire sans enfiler la tige par une extrémité dans le connecteur). Avant serrage, l'espace entre les branches est plus large que le diamètre de la tige qui peut donc être librement insérée. Ensuite, lorsqu'on serre les deux branches 16 en direction l'une de l'autre, l'empreinte 74 de la branche supérieure vient en contact avec la tige secondaire 3 qui se trouve ainsi en contact surfacique avec chacune des deux empreintes qui réalisent un blocage à friction de la tige secondaire 3 par rapport au connecteur 6, lesquels sont ainsi rigidement fixés l'un à l'autre.

La tige secondaire 3 est placée dans l'empreinte 74 de la branche inférieure après que la vis secondaire 12 a été introduite dans l'orifice 52. La position de l'empreinte 74 de la branche inférieure fait en sorte que la tige secondaire 3 s'étend alors dans le trajet de la tête de la vis secondaire 12 pour son désengagement du connecteur et sa sortie de l'orifice 52. Par conséquent, une fois la tige secondaire 3 fixée au connecteur, la vis secondaire 12 ne peut plus être séparée du connecteur.

La branche supérieure 16 du connecteur présente à son extrémité libre une encoche 76 qui mord sur l'empreinte 74 à laquelle elle est contiguë et facilite la manoeuvre au moyen d'un outil de la vis de serrage 12 en dépit de l'encombrement de la branche supérieure.

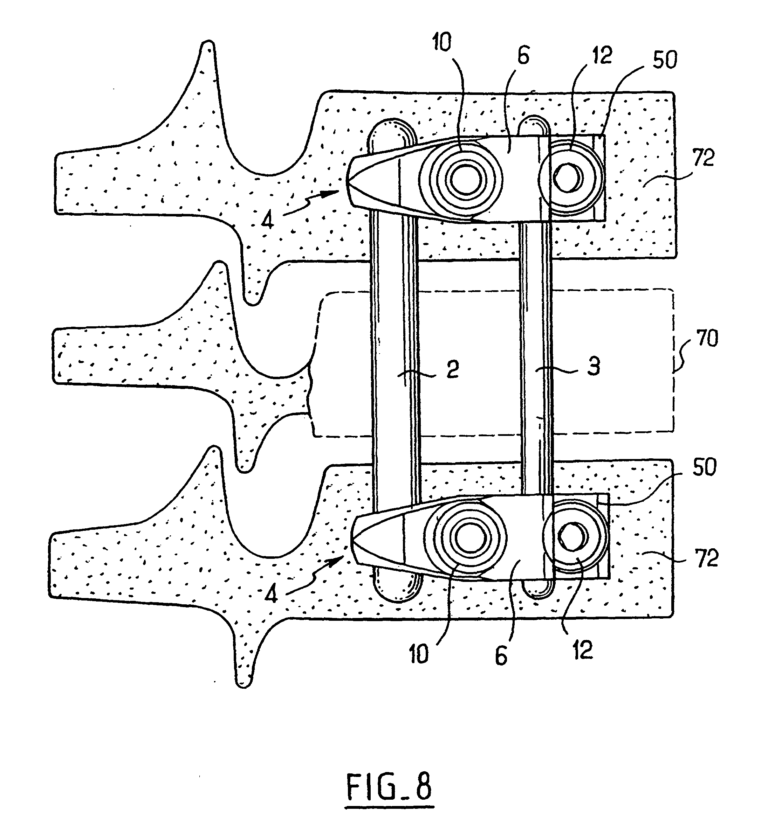

La mise en place d'un tel dispositif s'effectue de la manière suivante en référence à la figure 8 : après exposition de la vertèbre affectée 70 et des deux vertèbres adjacentes 72, on réalise une corporectomie tout en préservant, quand cela est possible, les plateaux respectifs de celles-ci. Pour chaque sous-ensemble 4, on réalise un avant-trou sur le côté latéral de la vertèbre 72 associée à égale distance des plateaux supérieur et inférieur, ainsi qu'à la limite du quart le plus postérieur du corps vertébral. On insère ensuite la vis principale 8 dans cet avant-trou jusqu'à la collerette d'arrêt 36. Le connecteur 6 est ensuite positionné sur la vis principale 8, bloqué en translation par la portée conique 38 de la vis principale 8 venant s'adapter à l'empreinte 40 du connecteur 6. On repère l'adaptation de la branche inférieure 16 du connecteur 6 sur la vertèbre, que l'on peut ajuster par le retrait du connecteur pour cintrer plus ou moins la branche inférieure 16 qui est sa partie la plus antérieure.

On vient ensuite visser la vis secondaire 12 par rapport à la vis principale 8 dans le second orifice 52 de la branche inférieure 16 jusqu'au contact du logement sphérique 60 du prolongement prévu à cet effet avec la partie sphérique 58 de la vis secondaire 12. Il est souhaitable de positionner le connecteur 6 le plus parallèle possible aux plateaux.

Après avoir instrumenté les deux vertèbres adjacentes 72, on positionne la tige principale 2 en l'enfilant par une extrémité dans les bagues 13 des connecteurs 6 et on règle la position angulaire de chaque sous-ensemble 4 par rapport à cette tige 2. On introduit ensuite latéralement la tige secondaire 3 dans les empreintes 74 des connecteurs 6 après l'avoir préalablement cintrée manuellement pour obtenir la courbure requise à l'étage rachidien correspondant. En cas de défaut, cette tige 3 peut être ôtée latéralement pour corriger sa courbure puis replacée. La figure 1 montre le système avant serrage des branches. Le serrage final s'opère grâce à la vis de serrage 10 qui s'insère dans la vis principale 8 et comprime ainsi le connecteur 6 pour serrer ses deux branches 16 l'une vers l'autre. Durant ce serrage, l'effort de serrage est dirigé d'abord sur la tige principale 2 via la bague 13, jusqu'à ce que l'empreinte 74 de la branche supérieure vienne en contact avec la tige secondaire 3. Dès lors, l'effort de serrage est réparti sur les deux tiges 2, 3. Ainsi, la réaction au niveau du couple vis principale 8/vis de serrage 10 est sensiblement coaxiale à celles-ci.

On pourra indépendamment de la présence du prolongement 50 et de la vis secondaire 12, mettre en oeuvre les caractéristiques relatives à l'association de la première vis 8 avec la vis de serrage 10.

Bien que cela soit moins avantageux, la branche prolongée pourra être celle destinée à être la plus distante de la vertèbre.

## Revendications

1. Système d'ostéosynthèse rachidienne, comprenant au moins un organe d'ancrage (8, 12), un élément de liaison allongé (2) et un connecteur (6) apte à être fixé à l'organe d'ancrage (8, 12) et à l'élément de liaison (2) en choisissant une position angulaire de l'élément de liaison par rapport au connecteur, **caractérisé en ce que** le système, notamment pour fixation antérieure, comprend un deuxième élément de liaison allongé (3), le connecteur (6) étant apte à être fixé simultanément aux deux éléments de liaison (2, 3), le système étant agencé de sorte que le deuxième élément de liaison (3) ne peut être fixé au connecteur (6) que dans une position angulaire par rapport au connecteur.

2. Système d'ostéosynthèse selon la revendication 1 **caractérisé en ce que** le deuxième élément de liaison (3) présente une résistance au cintrage plus faible que le premier élément de liaison (2).

3. Système d'ostéosynthèse selon l'une quelconque des revendications 1 à 2, **caractérisé en ce que** le connecteur (6) comporte deux branches (16) aptes à serrer au moins l'un des deux éléments de liaison (2, 3).

4. Système d'ostéosynthèse selon la revendication 3, **caractérisé en ce que** les branches présentent des premières extrémités par lesquelles elles sont reliées l'une à l'autre et des deuxièmes extrémités libres.

5. Système d'ostéosynthèse selon la revendication 4, **caractérisé en ce que** le connecteur est apte à recevoir le deuxième élément de liaison par insertion latérale entre les extrémités libres.

6. Système d'ostéosynthèse selon l'une quelconque des revendications 3 à 5, **caractérisé en ce que** les branches (16) sont aptes à serrer simultanément les deux éléments de liaison (2, 3).

7. Système d'ostéosynthèse selon l'une quelconque des revendications 3 à 6, **caractérisé en ce que** l'organe d'ancrage (8, 12) comprend une vis vertébrale, au moins une première des deux branches (16) étant apte à être engagée sur la vis (8, 12).

8. Système d'ostéosynthèse selon la revendication 7, **caractérisé en ce que** la vis vertébrale (8) présente un orifice fileté (39), le système comprenant une vis de serrage (10) apte à constituer une liaison vis-écrou avec cet orifice et à prendre appui sur une des branches (16) pour serrer les branches.

9. Système d'ostéosynthèse selon la revendication 7 ou 8, **caractérisé en ce qu'**il comprend une deuxième vis vertébrale (12), la première branche (16) étant apte à être engagée simultanément sur les deux vis vertébrales (8, 12).

10. Système d'ostéosynthèse selon la revendication 9, **caractérisé en ce qu'**il est agencé de sorte que le deuxième élément de liaison (3), lorsqu'il est fixé au connecteur (6), s'étend dans une trajectoire de la deuxième vis vertébrale (12) pour son désengagement du connecteur (6).

11. Système d'ostéosynthèse selon l'une quelconque des revendications 3 à 10, **caractérisé en ce qu'**il comprend une bague (13) apte à être enfilée sur le premier élément de liaison (2) et reçue entre les branches (16) pour choisir la position angulaire du premier élément de liaison (2) avant serrage des branches.

## Patentansprüche

1. Wirbelsäulen-Osteosynthesesystem, das mindestens ein Verankerungsorgan (8, 12), ein längliches Verbindungselement (2) und ein Anschlußstück (6) umfaßt, das dazu eingerichtet ist, am Verankerungsorgan (8, 12) und am Verbindungselement (2) befestigt zu werden, indem man eine Winkelposition des Verbindungselements bezüglich des Anschlußstücks wählt, **dadurch gekennzeichnet, dass** das System, besonders zur vorhergehenden Befestigung, ein zweites, längliches Verbindungselement (3) aufweist, wobei das Anschlußstück (6) dazu eingerichtet ist, gleichzeitig an den beiden Verbindungselementen (2, 3) befestigt zu werden, und dass das System derart ausgestaltet ist, dass das zweite Verbindungselement (3) nur in einer Winkelposition bezüglich des Anschlußstücks am Anschlußstück (6) befestigt werden kann.

2. Osteosynthesesystem nach Anspruch 1, **dadurch gekennzeichnet, dass** das zweite Verbindungselement (3) einen geringeren Krümmungswiderstand aufweist als das erste Verbindungselement (2).

3. Osteosynthesesystem nach irgendeinem der Ansprüche 1 oder 2, **dadurch gekennzeichnet, dass** das Anschlußstück (6) zwei Schenkel (16) aufweist, die dazu eingerichtet sind, mindestens eines der beiden Verbindungselemente (2, 3) festzuspannen.

4. Osteosynthesesystem nach Anspruch 3, **dadurch gekennzeichnet, dass** die Schenkel erste Enden, durch die sie miteinander verbunden sind, und zweite, freie Enden aufweisen.

5. Osteosynthesesystem nach Anspruch 4, **dadurch gekennzeichnet, dass** das Anschlußstück dazu eingerichtet ist, das zweite Verbindungselement durch seitliches Einschieben zwischen die freien Enden aufzunehmen.

6. Osteosynthesesystem nach irgendeinem der Ansprüche 3 bis 5, **dadurch gekennzeichnet, dass** die Schenkel (16) dazu eingerichtet sind, gleichzeitig die beiden Verebindungselemente (2, 3) festzuspannen.

7. Osteosynthesesystem nach irgendeinem der Ansprüche 3 bis 6, **dadurch gekennzeichnet, dass** das Verankerungsorgan (8, 12) eine Wirbelschraube aufweist, und dass mindestens ein erster der beiden Schenkel (16) dazu eingerichtet ist, mit der Schraube (8, 12) in Eingriff zu gelangen.

8. Osteosynthesesystem nach Anspruch 7, **dadurch gekennzeichnet, dass** die Wirbelschraube (8) eine Gewindeöffnung (39) aufweist, und dass das System eine Festspannschraube (10) aufweist, die dazu eingerichtet ist, eine Schrauben-Mutter-Verbindung mit dieser Öffnung herzustellen und auf einem der Schenkel (16) aufzusitzen, um die Schenkel festzuspannen.

9. Osteosynthesesystem nach Anspruch 7 oder 8, **dadurch gekennzeichnet, dass** es eine zweite Wirbelschraube (12) aufweist, und dass der erste Schenkel (16) dazu eingerichtet ist, gleichzeitig mit den beiden Wirbelschrauben (8, 12) in Eingriff zu stehen.

10. Osteosynthesesystem nach Anspruch 9, **dadurch gekennzeichnet, dass** es derart ausgebildet ist, dass das zweite Verbindungselement (3), wenn es am Anschlußstück (6) befestigt ist, sich in einer Bewegungsbahn der zweiten Wirbelschraube (12) erstreckt, und zwar für sein Lösen vom Anschlußstück (6).

11. Osteosynthesesystem nach irgendeinem der Ansprüche 3 bis 10, **dadurch gekennzeichnet, dass** es einen Ring (13) aufweist, der dazu eingerichtet ist, auf dem ersten Verbindungselement (2) aufgezogen zu werden und zwischen den Schenkeln (16) aufgenommen zu werden, um die Winkellage des ersten Verbindungselements (2) vor dem Festspannen der Schenkel zu wählen.

## Claims

1. Spinal osteosynthesis system, comprising at least one anchoring member (8, 12), an elongate connection element (2) and a connector (6) which is able to be fixed to the anchoring member (8, 12) and to the connection element (2) by choosing an angular position of the connection element relative to the connector, **characterized in that** the system, in particular for anterior fixation, comprises a second elongate connection element (3), the connector (6) being able to be fixed simultaneously to the two connection elements (2, 3), the system being designed in such a way that the second connection element (3) can be fixed to the connector (6) only in one angular position relative to the connector.

2. Osteosynthesis system according to Claim 1, **characterized in that** the second connection element (3) has less resistance to bending than the first connection element (2).

3. Osteosynthesis system according to either one of Claims 1 and 2, **characterized in that** the connector (6) comprises two branches (16) which can clamp at least one of the two connection elements (2, 3).

4. Osteosynthesis system according to Claim 3, **characterized in that** the branches have first ends via which they are connected to one another and second free ends.

5. Osteosynthesis system according to Claim 4, **characterized in that** the connector is able to receive the second connection element by lateral insertion between the free ends.

6. Osteosynthesis system according to any one of Claims 3 to 5, **characterized in that** the branches (16) are able to simultaneously clamp the two connection elements (2, 3).

7. Osteosynthesis system according to any one of Claims 3 to 6, **characterized in that** the anchoring member (8, 12) comprises a vertebral screw, at least a first of the two branches (16) being able to be engaged on the screw (8, 12).

8. Osteosynthesis system according to Claim 7, **characterized in that** the vertebral screw (8) has a threaded orifice (39), the system comprising a clamping screw (10) which can constitute a screw-nut connection with this orifice and is able to bear on one of the branches (16) in order to clamp the branches.

9. Osteosynthesis system according to Claim 7 or 8, **characterized in that** it comprises a second vertebral screw (12), the first branch (16) being able to be engaged simultaneously on the two vertebral screws (8, 12).

10. Osteosynthesis system according to Claim 9, **characterized in that** it is designed in such a way that the second connection element (3), when fixed to the connector (6), extends in a trajectory of the second vertebral screw (12) for its disengagement from the connector (6).

11. Osteosynthesis system according to any one of Claims 3 to 10, **characterized in that** it comprises a ring (13) which can be engaged on the first connection element (2) and received between the branches (16) in order to choose the angular position of the first connection element (2) before the branches are clamped.
